# EUROPÄISCHE PATENTSCHRIFT

(11) **EP 3 100 007 B1**
(45) Veröffentlichungstag und Bekanntmachung des Hinweises auf die Patenterteilung: **13.12.2017**
(21) Anmeldenummer: 15702192.4
(22) Anmeldetag: 23.01.2015
(51) Int. Cl.: G01D 9/00

(54) **DATENLOGGER**
DATA LOGGER
ENREGISTREUR DE DONNÉES

(30) Priorität: 27.01.2014 DE 102014100920
(43) Veröffentlichungstag der Anmeldung: 07.12.2016
(73) Patentinhaber: XYLEM Analytics Germany GmbH, 82362 Weilheim (DE)
(72) Erfinder: BAUMANN, Michael, 86633 Neuburg (DE)
(74) Vertreter: Schlief, Thomas P.
(86) Internationale Anmeldenummer: PCT/EP2015/051322
(87) Internationale Veröffentlichungsnummer: WO 2015/110565

(56) Entgegenhaltungen:
- CH-B1- 698 757
- DE-B3-102011 054 838
- DE-U1-202008 014 764

## Beschreibung

Die vorliegende Erfindung betrifft einen Datenlogger nach dem Oberbegriff des Anspruchs 1.

Ein gattungsgemäßer Datenlogger zur Erfassung, Auswertung und Darstellung von aus Messwerten, beispielsweise Temperatur-Messwerten, erzeugten Daten ist aus der DE 10 2011 054 838 A1 bekannt. Dieser Datenlogger weist eine Sensoreinheit, eine Speichereinheit und eine Prozessoreinheit auf, die mit den anderen Einheiten zur Messwertaufnahme und zur Auswertung, Darstellung und Abspeicherung der Daten in Verbindung steht, wobei alle Geräteeinheiten in einem gemeinsamen Gehäuse angeordnet sind. Am Gehäuse ist eine USB-Schnittstelle für den digitalen Datenaustausch mit einem externen Computer vorgesehen.

Verwendung finden derartige Datenlogger insbesondere dort, wo eine Langzeitaufnahme von Messparametern vorgenommen werden soll, beispielsweise bei der Überwachung von Transporten, wobei insbesondere Temperatur- und Feuchtedaten, aber auch Beschleunigungs- und/oder Vibrationsdaten, von besonderem Interesse sein können. Auch können solche Daten zur Kontrolle der Bedingungen in Lagerhäusern (Temperatur, Feuchte), zur Fehleranalyse von Systemen, zur Überwachung und Alarmierung bei Produktionsprozessen und bei Qualitätsstudien sowie bei der Aufzeichnung von Daten im Hobbybereich, wie beispielsweise die Erfassung von Flugdaten eines Modellflugzeuges, verwendet werden.

Demnach können mit einem Datenlogger in Abhängigkeit von der Sensorik die unterschiedlichsten Messdaten über einen längeren Zeitraum erfasst und abgespeichert werden. Bei Bedarf können diese weiterverarbeitet, graphisch dargestellt und zur Dokumentation aufbereitet werden.

Eine häufige Anwendung von Datenloggern besteht insbesondere darin, dass ein solcher Datenlogger am Aufgabeort eines Containers oder eines anderen Frachtmittels programmiert und zum Transportgut gelegt wird. Am Zielort werden die während des Transports im Datenlogger gespeicherten Daten zur Temperatur und/oder Feuchte etc. dann ausgewertet und auf Fehler analysiert. Häufig müssen auch die entsprechenden Dateien an den Kunden der Spedition per E-Mail übersandt werden, um nachzuweisen, dass die Waren ohne Zwischenfälle (beispielsweise zu großer Temperaturanstieg bei gekühlter Ware, beispielsweise Pharmazeutika oder tiefgekühlte Lebensmittel) ihren Bestimmungsort erreicht hat.

Sowohl das direkte Auslesen von Messdaten als auch eine Programmierung und Konfiguration des Datenloggers mittels einer speziellen Software (z.B. zur Einstellung des Messtaktes und/oder der erlaubten Temperaturgrenzen) wird beim Anschluss des Datenloggers an den Computer durchgeführt. Zu diesem Zweck ist beispielsweise bei dem Datenlogger gemäß der DE 10 2011 054 838 A1 die USB-Schnittstelle des Loggers als Mehrklassenschnittstelle ausgebildet, welche den Datenlogger beim Verbinden mit dem externen Computer gleichzeitig mit zumindest zwei Schnittstellenklassen anmeldet. Somit ist der Datenlogger vom Computer im Wesentlichen synchron mittels der ersten Schnittstellenklasse als Massenspeicher und mittels der zweiten Schnittstellenklasse als Kommunikationsgerät für eine spezielle Auswerte- und/oder Programmiersoftware erkennbar.

Bekannte Datenlogger stellen die erfassten und gespeicherten Messdaten für gewöhnlich in einem PDF-Dokument zur Verfügung. Selbstverständlich ist auch jegliches andere Standardformat denkbar. Hierbei meldet sich der Datenlogger beim Anschluss an einen externen Computer, worunter im Rahmen dieser Erfindung generell alle Datenverarbeitungsanlagen subsumiert sein sollen (einschließlich tragbarer Geräte und Smart Phones etc.), als besagter Massenspeicher an. Für den Benutzer wird somit der Datenlogger auf dem externen Computer als Laufwerk erkennbar, auf dem die Messdaten in dem entsprechenden Format, beispielsweise im PDF-Format, gespeichert sind. Zum Übermitteln der im Datenlogger abgespeicherten Daten an einen Empfänger (Speditionszentrale, Versicherung, Auftraggeber, etc.) können die entsprechenden Daten entweder an eine vom Bediener am Zielort zu erstellende E-Mail angehängt werden, oder auf dem externen Computer ist eine spezielle Firmensoftware gespeichert, welche das Versenden der Daten automatisch durchführt. Alternativ können die Daten in entsprechende Verzeichnisse des Firmennetzwerks (Intranet) oder in einen Internet-Speicherplatz (Cloud) kopiert werden.

Aus der DE 20 2008 014 764 U1 ist ein Sensor bekannt, bei dem ein zweites Anschlussmittel zum Anschließen des Sensors an einen externen Rechner für einen Konfigurationsmodus vorhanden ist. In einem Speicher der Steuer- und Auswerteeinheit des Sensors ist eine Konfigurationssoftware gespeichert, welche in einen Arbeitsspeicher eines externen Rechners geladen werden kann, wenn der Sensor an den externen Rechner angeschlossen ist. Die Konfigurationssoftware ist zum automatischen oder interaktiven Konfigurieren des Sensors durch einen Benutzer eingerichtet.

Aus der CH 698 757 B1 ist ein Datenlogger zur Erfassung, Auswertung und Darstellung von aus Messwerten erzeugten Klimadaten bekannt. Der Datenlogger umfasst eine Erfassungseinheit, die nach einem vorgebbaren Schema Messdaten aufnimmt und über eine eigenständige Energieversorgung verfügt. Des Weiteren umfasst der Datenlogger eine Auswerteeinheit, die zur Verarbeitung der Messdaten und zur Darstellung der aus den Messdaten gewonnenen Klimadaten in Reportform in einem PDF-Format ausgebildet ist. Die Auswerteeinheit, die Speichereinheit und die Steuereinheit sind über eine am Gehäuse vorgesehene Schnittstelle mit Energie versorgbar und aktivierbar. Die Auswerteeinheit umfasst einen Mikroprozessor, der derart ausgebildet ist, dass er aktivierbar ist, wenn der Datenlogger über seinen als eine USB-Schnittstelle ausgebildeten Ausgang an einen externen Rechner angedockt und mit externer Energie versorgt worden ist. Der Prozessor wandelt die Dateien dabei in ein international standardisiertes PDF-Format um, welches über eine weltweit verfügbare Standardsoftware lesbar ist. Zusätzlich ist der Prozessor dazu ausgebildet, die digitalen Eingangs- und Zeitdaten sowie die Ereignisdaten als geschützte Rohdaten derart in das erzeugte PDF-Datenformat einzubetten, dass diese nur mit einer gerätespezifischen Auswertesoftware les- und bearbeitbar sind. Dazu sind die Rohdaten in einen geschlossenen, geschützten Bereich des PDF-Datenfiles eingebettet.

Nachteilig bei dem vorstehend genannten Stand der Technik ist es, dass der Benutzer weitere Arbeitsschritte, die der Datenkonvertierung nachgelagert sind, manuell vornehmen muss.

Aufgabe der vorliegenden Erfindung ist es, einen Datenlogger mit erhöhtem Bedienkomfort zu schaffen.

Die Aufgabe wird gelöst durch einen Datenlogger mit den Merkmalen des unabhängigen Patentanspruchs 1.

Die Vorteile der Erfindung sind insbesondere darin zu sehen, dass - gemäß einer ersten Alternative - die Prozessoreinheit nicht nur zur Abspeicherung von digitalen Daten in der Speichereinheit ausgebildet ist, sondern auch eine ausführbare Datei (insbesondere als EXE-Datei) zu erzeugen vermag. Gemäß einer zweiten Alternative ist eine solche ausführbare Datei schon im Datenlogger abgespeichert, wobei die Übertragung dieser Datei vorzugsweise beim Programmieren des Datenloggers (Messtakt, Temperaturbereich, Alarmgrenzwert, ...) erfolgt. Wird der erfindungsgemäße Datenlogger zum Überwachen der Umgebungsbedingungen während eines Transports von Waren (beispielsweise von Pharmazeutika) eingesetzt, wird die genannte ausführbare Datei beim Verbinden mit einem externen Computer, üblicherweise am Zielort, als eine solche ausführbare Datei angezeigt, die mit einer Standardsoftware ausführbar ist. Eine solche Standardsoftware ist beispielsweise bei einem Computer mit einem Microsoft-Betriebssystem stets vorhanden. Die Anzeige der ausführbaren Datei auf einem mit dem externen Computer verbundenen Bildschirm erfolgt beispielsweise, indem der Datenlogger beim Anschließen an den Computer automatisch als Laufwerk angezeigt wird, welches durch Anklicken die auf dem Datenlogger befindlichen Dateien anzeigt, unter denen sich die genannte ausführbare Datei befindet. Wird der Datenlogger nicht automatisch als Laufwerk angezeigt, ist die Dateistruktur zu öffnen (bei einem Microsoft-Betriebssystem mittels dem "Explorer"), woraufhin das Laufwerk des Datenloggers angezeigt wird.

Beim Ausführen der ausführbaren Datei, bekanntermaßen durch Anklicken der Datei, wird - in Verbindung mit einem auf dem externen Computer installiertem E-Mail Programm (z.B. Microsoft Outlook) - eine E-Mail auf dem externen Computer generiert.

Gemäß einer vorteilhaften Ausführungsform der Erfindung wird die E-Mail auf dem Bildschirm des externen Computers angezeigt. Zu dieser E-Mail wird automatisch, d.h. beim Ausführen des Programms durch Anklicken, mindestens eine der folgenden Aktionen ausgeführt:
- Einfügen einer Empfängeradresse in die E-Mail,
- Einfügen einer Absenderadresse in die E-Mail,
- Einfügen eines Betreffs in die E-Mail,
- Einfügen eines Inhalts in die E-Mail, und/oder
- Anhängen einer von der Prozessoreinheit erzeugten Datei aus den in der Speichereinheit gespeicherten Daten.

Die zum Einfügen notwendigen Informationen (Empfängeradresse, Absenderadresse, Betreff, Inhalt) holt sich das ausführbare Programm aus im Datenlogger gespeicherten Daten. Vorzugsweise wurden diese zuvor in einem entsprechenden Speicherort im Datenlogger abgelegt, vorzugsweise beim Programmieren des Loggers am Startort des Transports.

Alternativ oder zusätzlich kann die ausführbare Datei so konfiguriert werden, dass die von der Prozessoreinheit erzeugten Dateien automatisch beim Ausführen des Programms in ein definiertes Verzeichnis eines Computers, Servers oder Internet-Speicherortes (z.B. Cloud) kopiert werden. Weiterhin kann vorteilhafterweise vorgesehen sein, dass die ausführbare Datei derart ausgebildet ist, dass sie das Erstellen und Versenden einer E-Mail an einen vordefinierten Empfänger veranlasst, mit welcher der Empfänger über die Abspeicherung der mindestens einen Datei aus der besagten Speichereinheit des Datenloggers in dem genannten externen Speichermedium informiert wird.

Die erfindungsgemäße Ausgestaltung ermöglicht es, dass auch ohne eine spezielle Firmensoftware und/oder ohne eine spezielle Schulung von Bedienpersonal eine äußerst einfache, nicht oder in wesentlich geringerem Maße fehleranfällige und damit sichere Übertragung von Daten aus dem Logger gewährleistet wird. Da keine firmenspezifische Software notwendig ist, entfällt eine entsprechende Installation, die an den unterschiedlichsten geografischen Orten unter Antreffen diversester Bedingungen bei den dort verwendeten Computern durchzuführen wäre. Da nie garantiert werden kann, dass solche Installationen auf den vorhandenen Computern fehlerfrei gelingen oder das entsprechende Personal für solche Installationen evtl. gar nicht vorhanden ist, ist es von großem Vorteil, wenn auf eine Installation von firmenspezifischer Software gänzlich verzichtet werden kann.

Gleichfalls wird der Fehleraufwand reduziert, indem - gemäß einer der bevorzugten Ausführungsformen der Erfindung (s.o.) - eine vorzugsweise vollautomatische E-Mail-Erstellung durch Anklicken und Ausführen der ausführbaren Datei einschließlich des Ausfüllens von E-Mail-Angaben und/oder Anhängen der die Messdaten erhaltenen Datei(en) an diese E-Mail realisiert wird. Der Vorteil liegt hierbei darin, dass die Fehleranfälligkeit deutlich reduziert wird, da eine Bedienperson weniger bis keine Fehleingaben tätigen bzw. falsche (oder sogar gefälschte) Dateien anhängen kann.

Der erfindungsgemäße Automatisierungsgrad durch automatisches Erzeugen einer E-Mail mit bevorzugt allen relevanten Informationen (Absender, Empfänger, Betreff, Inhalt, Dateienanhang) erfordert im einfachsten Fall lediglich das Anschließen des Datenloggers an einen mit dem Internet verbundenen Computer (auf dem lediglich ein E-Mail-Programm installiert sein muss, das ohne Weiteres ein Standard-E-Mail-Programm sein kann), das Öffnen des sich als Laufwerk am Computer angemeldeten Datenlogger-Laufwerks sowie das Anklicken der sich auf diesem Laufwerk befindlichen ausführbaren Datei. Gemäß der am meisten bevorzugten Ausführungsform der Erfindung veranlasst die Datei das Öffnen des E-Mail-Programms auf dem externen Computer und anschließend die Erzeugung einer E-Mail und das automatische Ausfüllen der wesentlichen Informationen in dieser E-Mail sowie das automatische Anhängen von einer oder mehreren von der Prozessoreinheit erzeugten Dateien, welche sich auf die digitalen Messdaten beziehen und weitere Daten umfassen können. Die E-Mail ist dann lediglich noch aktiv vom Bediener, vorzugsweise über das Internet, abzusenden, wobei aber auch dieser Schritt automatisiert ablaufen könnte. Der Empfänger, der die Datei(n) erhält, kann diese dann auswerten, analysieren, archivieren, Dritten zur Verfügung stellen (Spedition, Kunde, Versicherung etc.). Der oder die Empfängeradressen in der E-Mail können sich beispielsweise auf eine Firmenadresse und/oder eine Adresse in der Cloud beziehen.

Vorzugsweise ist die Prozessoreinheit derart ausgebildet, dass sie digitale Daten, insbesondere digitale Messdaten in Roh- oder aufbereiteter Form, in der Speichereinheit in Form einer PDF-Datei und in einem zweiten Dateiformat, beispielsweise als ED3-Datei, abzuspeichern vermag. Mindestens eine, vorzugsweise beide Dateien werden vorzugweise automatisch beim Ausführen der genannten ausführbaren Datei an die besagte E-Mail angehängt.

Gemäß einer vorteilhaften Ausführungsform ist die Prozessoreinheit derart ausgebildet, dass sie in der besagten PDF-Datei aufbereitete Daten, welche beispielsweise tabellarisch oder graphisch die Messdaten (z.B. T-Messwerte) und die zugehörigen Messzeiten sowie ggf. einen entsprechenden Graphen beinhalten, abzuspeichern vermag. Auch können andere Ereignisdaten in der PDF-Datei abgelegt werden. Diese PDF-Datei dient zur Veranschaulichung des Einhaltens oder Abweichens der Bedingungen während des Transports und kann weitere Daten wie Informationen hinsichtlich des Transportguts und der Transportmenge, des Transportzeitraums und des Transportwegs, des Spediteurs, etc. beinhalten.

Die oben genannte Erzeugung einer PDF-Datei hat zusätzlich den Vorteil, dass die Daten, nach Anstecken des Datenloggers an einen externen Computer, mittels frei erhältlicher Standardsoftware lesbar sind (beispielsweise dem verbreiteten Adobe® Reader) und auf den externen Computer übertragbar sind.

Alternativ, vorzugsweise zusätzlich ist die Prozessoreinheit derart ausgebildet, dass sie eine eigene Datei in einem anderen Format, beispielsweise vom ED3-Typ, mit den digitalen bzw. digitalisierten Messdaten als Rohdaten erzeugt, wobei diese Datei - vorzugsweise automatisch durch Anklicken der ausführbaren Datei - ebenfalls an die besagte E-Mail angehängt wird.

Mit besonderem Vorteil ist die Prozessoreinheit mittels einer speziellen Auswerte- und/oder Programmiersoftware programmierbar und/oder konfigurierbar, insbesondere zur Abspeicherung der besagten E-Mail-Empfängeradresse, der besagten E-Mail-Absenderadresse, des besagten E-Mail-Betreffs und/oder des besagten E-Mail-Inhalts in einem Speicherort des Datenloggers. Dieser Speicherort kann beispielsweise Teil der besagten Speichereinheit sein.

Auch bei der weiteren Erfindungsvariante des automatischen Abspeicherns bzw. des Kopierens der mindestens einen Datei aus der besagten Speichereinheit des Datenloggers in ein externes Speichermedium ist es von Vorteil, wenn die Adresse bzw. der Pfad dieses besagten externen Speichermediums in einem Speicherort des Datenloggers abgelegt ist. Auch hier kann dieser Speicherort Teil der besagten Speichereinheit im Datenlogger sein.

Um eine Überprüfung des Messergebnisses auch ohne eine externe Datenverarbeitungsanlage durchführen zu können, ist es vorteilhaft, wenn der Datenlogger eine Anzeige, insbesondere ein Display, ein Bedienelement, insbesondere eine Taste und/oder einen Schalter, und/oder ein kombiniertes Anzeige-/Bedienelement, insbesondere einen Touchscreen, aufweist.

Weitere Vorteile der Erfindung sind in dem nachfolgenden Ausführungsbeispiel beschrieben. Es zeigt:
- **Fig. 1**: ein Blockschema des Datenloggers, angeschlossen an einen externen Computer, und
- **Fig. 2**: ein Flussdiagramm.

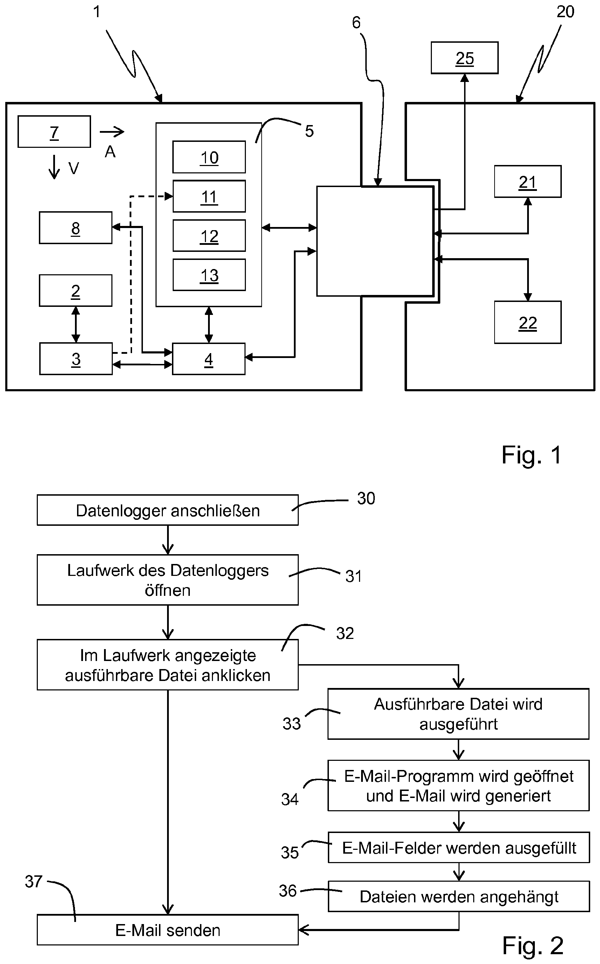

Figur 1 zeigt einen Datenlogger 1, der über eine Schnittstelle 6 mit einem externen Computer 20 verbunden ist. Die Schnittstelle 6 ist vorzugsweise als USB-Schnittstelle oder aber als Firewire-Schnittstelle ausgebildet. Zum Erfassen, Speichern, Auswerten, Darstellen und/oder Auslesen von Messdaten weist der Datenlogger 1 eine Sensoreinheit 2, vorliegend insbesondere ausgebildet zur Erfassung der Temperatur, einen Analog/Digital-Konverter 3 zur Umwandlung der gemessenen analogen Messdaten in digitale Messdaten, eine Prozessoreinheit 4 sowie eine Speichereinheit 5 auf. Mittels der Sensoreinheit 2 werden Informationen, insbesondere die Temperatur, aus der Umgebung über einen längeren Zeitraum erfasst und kontinuierlich an die Prozessoreinheit 4 weitergeleitet. Diese bereitet die erfassten Messdaten derart auf, dass sie insbesondere graphisch darstellbar sind.

Die Rohdaten und/oder die aufbereiteten Daten werden von der Prozessoreinheit 4 vorzugsweise als PDF-Datei 10 in der Speichereinheit 5 abgespeichert. In der PDF-Datei 10 sind die aufbereiteten Messdaten (zusammen mit den dazugehörigen Zeitdaten) in tabellarischer und/oder grafischer Form sowie ggf. Ereignisdaten (Batteriewechsel, Bedienereingriffe, Transportweg, Fracht, etc.) enthalten.

In einer weiteren Datei 11 der Speichereinheit 5 sind ausschließlich die sensiblen Rohdaten der Messreihe abgespeichert. Diese Rohdaten können auch, ohne dass sie die Prozessoreinheit 4 durchlaufen, als Datei 11 in der Speichereinheit 5 abgespeichert werden (s. gestrichelte Linie). Nicht dargestellt ist zudem ein optionaler Zeitgeber, der zu jedem Messsignal das zugehörige Datum und die zugehörige Zeit erfasst und diese Information (ggf. über einen Pufferspeicher, in dem auch die Messdaten vorübergehend abgelegt sein können) an die Speichereinheit 5 weiterleitet. Alternativ ist es möglich, dass die Messdaten immer in gleichen Abständen erfasst werden, so dass kein Zeitgeber vonnöten ist. Die Datei für die Rohdaten kann insbesondere vom ED3-Format sein.

Eine Energieversorgungsquelle 7, beispielsweise in Form einer Blockbatterie, versorgt die verschiedenen Einheiten des Datenloggers 1, insbesondere die Sensoreinheit 2 und die Prozessoreinheit 4, mit der notwendigen Energie.

Um während oder nach der Messung eine Überprüfung des Messergebnisses durchführen zu können, weist der Datenlogger 1 ferner eine Anzeige-/Bedieneinheit 8 auf. Diese besteht für gewöhnlich aus einem hier nicht dargestellten Display und einem oder mehreren Schaltern oder Knöpfen. Ferner kann die Anzeigebedieneinheit 8 als Touchscreen ausgebildet sein. Ein Benutzer kann somit während oder nach der Messung eine Überprüfung der vorzugsweise graphisch aufbereiteten Messdaten vornehmen. Ferner können auch nach der Messung die in der PDF-Datei 10 abgelegten, aufbereiteten Messdaten eingesehen werden. Des Weiteren kann über der Anzeige-/Bedieneinheit 8 interagiert werden, um beispielsweise die Messung zu starten oder zu stoppen.

Der Datenlogger 1 weist vorteilhafterweise ein Gehäuse auf, in dem die besagten Einheiten 2-8 integriert sind. Es können aber auch weitere Schnittstellen am Datenlogger 1 vorgesehen sein, an denen beispielsweise externe Sensoreinheiten anschließbar sind. Es ist hierbei prinzipiell möglich, dass in dem Gehäuse keine Sensoreinheit vorgesehen ist.

In der Fig. 1 ist dargestellt, wie der Datenlogger 1 an einen externen Computer 20 angeschlossen ist. Dieser besitzt ein Betriebssystem 21, beispielsweise ein Windows-Betriebssystem von Microsoft. Außerdem ist ein E-Mail-Programm 22, beispielsweise Outlook von Microsoft, auf dem Computer installiert. Der externe Computer 20 ist zudem an das Internet angeschlossen.

Der Datenlogger 1 ist erfindungsgemäß derart ausgebildet, dass die Prozessoreinheit 4 gemäß einer ersten Variante eine ausführbare Datei 12 zu erzeugen vermag, die im vorliegenden Fall ebenfalls in der Speichereinheit 5 abgelegt wird. Gemäß einer anderen Variante muss erst keine solche ausführbare Datei 12 vom Datenlogger 1 erzeugt werden, sondern ist schon darin abgespeichert, wobei sich auch hier die Abspeicherung in der Speichereinheit 5 anbietet; diese ausführbare Datei 12 kann insbesondere vor dem Beigeben des Datenloggers 1 zu einem Warentransport am Aufgabeort mittels eines Computers in die Speichereinheit 5 gespeichert werden (nicht dargestellt).

Anhand der Fig. 2 wird ein möglicher Ablauf der Übermittlung der Dateien 10 und 11 an eine Zieladresse erläutert, damit die in der Speichereinheit 5 abgelegten Daten an einem anderen Ort als dem Zielort angesehen bzw. analysiert werden können.

Zuerst wird, vorzugsweise am Zielort des Warentransports, der mittransportierte Datenlogger 1 über die Schnittstelle 6 mit dem externen Computer 20 verbunden (Schritt 30 in Fig. 2). Je nach Einstellung des Computers 20 wird der Datenlogger 1 automatisch als neu angeschlossenes Laufwerk auf dem am Computer 20 angeschlossenen Bildschirm angezeigt, oder dieses Laufwerk wird manuell zur Anzeige auf dem Bildschirm gebracht (z.B. mit Hilfe des "Explorers" o.dgl.). Dieses angezeigte Laufwerk wird dann vom Bediener geöffnet (Schritt 31), woraufhin die auf dem Laufwerk befindliche ausführbare Datei 12 angezeigt wird und seitens des Bedieners angeklickt werden kann (Schritt 32). Durch das Anklicken wird die ausführbare Datei 12 vom Betriebssystem 21 des externen Computers 20 ausgeführt (Schritt 33). Die Ausführung der Datei 12 startet - falls noch nicht geschehen - das E-Mail-Programm 22. Zudem wird eine E-Mail in diesem E-Mail-Programm 22 generiert (Schritt 34), dessen Felder (Empfänger- und Absenderadressen, Betreff, Inhalt) automatisch ausgefüllt werden (Schritt 35). Hierzu greift das E-Mail-Programm 22 automatisch auf einen Speicherort 13 der Speichereinheit 5 des Datenloggers 5 zu, in welchem diese Angaben abgespeichert wurden, zweckmäßigerweise durch entsprechende Programmierung des Datenloggers 1 am Aufgabeort des Warentransports. Diese Programmierung erfolgt vorzugsweise zusammen mit der Programmierung des Datenloggers 1 hinsichtlich Messtakt, Messgrenzwerten, etc., welche über die Schnittstelle 6 erfolgt. Zusätzlich zum Übergeben der genannten Informationen an die E-Mail werden die Dateien 10, 11 an diese E-Mail angehängt (Schritt 36).

Die E-Mail wird dann lediglich noch vom Bediener versendet (Schritt 37). Die Dateien 10, 11 werden auf diesem Wege beispielsweise in einer webbasierten Cloud-Software gespeichert und stehen zur Ansicht und Verarbeitung zur Verfügung. Der E-Mail-Server kann auch beim Spediteur, bei dem Kunden oder an anderer Stelle vorgesehen sein. Die PDF-Datei 10 dient insbesondere der Visualisierung der Transportparameter, während die Datei 11, beispielsweise vorliegend im ED3-Format, die Rohdaten der Messungen wiedergibt.

Die in der linken Hälfte der Fig. 2 dargestellten Schritte sind gemäß dem diskutierten Beispiel vom Bediener auszuführen, während die Schritte in der rechten Hälfte automatisch ablaufen. Es ist aber auch ohne Weiteres möglich, dass noch weitere Schritte, ggf. sogar alle bis auf das Anschließen des Datenloggers 1 an den externen Computer 20, automatisch ablaufen.

In den Figuren ist nur der Fall einer einzigen Sensoreinheit dargestellt. Ohne Weiteres können auch mehrere Sensoreinheiten mit mehreren Analog/Digital-Konvertern vorgesehen sein.

Gemäß einer alternativen oder zusätzlichen Ausführungsform kann durch das Anklicken der ausführbaren Datei 12 auch die automatische Übertragung der Dateien 10, 11 aus der besagten Speichereinheit 5 in ein externes Speichermedium 25 veranlasst werden (s. Fig. 1). Dieses definierte Verzeichnis kann in einem Computer, einem Server oder einem Internet-Speicherort vorliegen. (z.B. Cloud). Auch kann die ausführbare Datei 12 derart ausgebildet sein, dass sie das Erstellen und Versenden einer E-Mail an einen vordefinierten Empfänger (beispielsweise abgespeichert im Speicherort 13) veranlasst, mit welcher dieser über die Abspeicherung der Dateien 10, 11 in dem genannten externen Speichermedium 25 informiert wird. Das vorgenannte Abspeichern bzw. Kopieren der Dateien 10, 11 in dem externen Speichermedium 25 sowie alle anderen Schritte einschließlich der E-Mail-Benachrichtigung können auch automatisch erfolgen, wenn lediglich der Datenlogger 1 am externen Computer 20 angeschlossen wird.

Die Erfindung ist sowohl für nur einmal und für mehrfach verwendbare Datenlogger einsetzbar.

Die vorliegende Erfindung ist nicht auf das dargestellte und beschriebene Ausführungsbeispiel beschränkt. Abwandlungen im Rahmen der Patentansprüche sind ebenso möglich wie eine Kombination der Merkmale auch, wenn sie in unterschiedlichen Ausführungsbeispielen beschrieben sind. So kann beispielsweise vorgesehen sein, dass die PDF-Datei 10 erst beim Anschließen des Datenloggers 1 an dem externen Computer 20 automatisch generiert wird.

### Bezugszeichenliste

- 1: Datenlogger
- 2: Sensoreinheit
- 3: Analog/Digital-Konverter
- 4: Prozessoreinheit
- 5: Speichereinheit
- 6: Schnittstelle
- 7: Energieversorgungsquelle
- 8: Anzeige-/Bedieneinheit
- 10: PDF-Datei
- 11: ED3-Datei
- 12: ausführbare Datei
- 13: Speicherort
- 20: externer Computer
- 21: Betriebssystem
- 22: E-Mail-Programm
- 25: externes Speichermedium
- 30-36: Abfolgeschritte

## Patentansprüche

1. Datenlogger (1) zum Erfassen, Speichern, Auswerten, Darstellen und/oder Auslesen von Messdaten mit mindestens den folgenden, vorzugsweise in einem gemeinsamen Gehäuse angeordneten Funktionseinheiten:
mindestens einer Sensoreinheit (2) zum Messen von Umgebungsparametern in Form von analogen Messwerten, wie insbesondere Temperatur und/oder Feuchte,
mindestens einem Analog/Digital-Konverter (3) zur Umwandlung der analogen Messwerte in digitale Messdaten,
mindestens einer Prozessoreinheit (4) zur Erzeugung mindestens einer Datei (10, 11) aus den digitalen Messdaten, ggf. in aufbereiteter Form,
mindestens einer Speichereinheit (5) zur Speicherung von digitalen Daten, insbesondere der mindestens einen Datei (10, 11), und
mindestens einer Schnittstelle (6), vorzugsweise einer USB-Schnittstelle, zur Übertragung von zumindest digitalen Daten zum und/oder vom Datenlogger (1), und
einer Energieversorgungsquelle (7),
**dadurch gekennzeichnet,**
**dass** die Prozessoreinheit (4) des Weiteren zur Erzeugung einer ausführbaren Datei (12) ausgebildet ist oder dass eine solche ausführbare Datei (12) schon im Datenlogger (1) abgespeichert ist,
wobei diese ausführbare Datei (12) beim Verbinden mit einem externen Computer (20) als mit dem Betriebssystem (21) des externen Computers (20) ausführbare Datei (12) angezeigt wird oder von diesem Betriebssystem (21) automatisch ausgeführt wird und
derart ausgebildet ist, dass beim Ausführen dieser Datei (12) über ein auf dem externen Computer (20) installiertes E-Mail-Programm eine E-Mail auf dem externen Computer (20) generiert wird.

2. Datenlogger nach Anspruch 1, **dadurch gekennzeichnet, dass** zu der E-Mail mindestens eine der folgenden Aktionen automatisch aus im Datenlogger (1) gespeicherten Daten ausgeführt wird:
- Einfügen einer Empfängeradresse in die E-Mail,
- Einfügen einer Absenderadresse in die E-Mail,
- Einfügen eines Betreffs in die E-Mail,
- Einfügen eines Inhalts in die E-Mail, und/oder
- Anhängen mindestens einer Datei (10, 11) aus der besagten Speichereinheit (5) des Datenloggers (1).

3. Datenlogger nach Anspruch 1 oder 2, **dadurch gekennzeichnet, dass** die ausführbare Datei (12) derart ausgebildet ist, dass beim Ausführen dieser Datei (12) mindestens eine Datei (10, 11) aus der besagten Speichereinheit (5) des Datenloggers (1) in einem externen Speichermedium (25) gespeichert wird.

4. Datenlogger nach Anspruch 3, **dadurch gekennzeichnet, dass** die ausführbare Datei (12) derart ausgebildet ist, dass sie das Erstellen und Versenden einer E-Mail an einen vordefinierten Empfänger veranlasst, mit welcher der Empfänger über die Abspeicherung der mindestens einen Datei (10, 11) aus der besagten Speichereinheit (5) des Datenloggers (1) in dem genannten externen Speichermedium (25) informiert wird.

5. Datenlogger nach einem oder mehreren der vorhergehenden Ansprüche, **dadurch gekennzeichnet, dass** die Prozessoreinheit (4) digitale Daten, insbesondere digitale Messdaten in Roh- oder aufbereiteter Form, in der Speichereinheit (5) in Form einer PDF-Datei (10) und in einer Datei (11) mit anderem Format, beispielsweise als ED3-Datei, abzuspeichern vermag, wobei mindestens eine dieser Dateien (10, 11) vorgesehen ist zum Anhängen an die besagte E-Mail, vorzugsweise automatisch beim Ausführen der besagten ausführbaren Datei (12), oder zum Abspeichern in einem externen Speichermedium (25).

6. Datenlogger nach einem oder mehreren der vorhergehenden Ansprüche, **dadurch gekennzeichnet, dass** die Prozessoreinheit (4) derart ausgebildet ist, dass sie in der PDF-Datei (10) aus den digitalen Messdaten aufbereitete Daten abzuspeichern vermag.

7. Datenlogger nach einem oder mehreren der vorhergehenden Ansprüche, **dadurch gekennzeichnet, dass** die Prozessoreinheit (4) derart ausgebildet ist, dass sie in der Datei (11) des anderen Formats die digitalen Messdaten im Rohformat abzuspeichern vermag.

8. Datenlogger nach einem oder mehreren der vorherigen Ansprüche, **dadurch gekennzeichnet, dass** die Prozessoreinheit (4) mittels einer speziellen Auswerte- und/oder Programmiersoftware programmierbar und/oder konfigurierbar ist, insbesondere zur Abspeicherung der besagten E-Mail-Empfängeradresse, der besagten E-Mail-Absenderadresse, des besagten E-Mail-Betreffs und/oder des besagten E-Mail-Inhalts und/oder der Adresse bzw. des Pfades des besagten externen Speichermediums (25) in einem Speicherort (13) des Datenloggers (1).

9. Datenlogger nach einem oder mehreren der vorherigen Ansprüche, **dadurch gekennzeichnet, dass** der Datenlogger (1) eine Anzeige, insbesondere ein Display, ein Bedienelement, insbesondere eine Taste und/oder Schalter, und/oder ein kombiniertes Anzeige-/Bedienelement (8), insbesondere einen Touchscreen, aufweist.

## Claims

1. Data logger (1) for recording, storing, evaluating, displaying and/or reading measurement data with at least the following functional units, preferably arranged in a common housing:
at least one sensor unit (2) for measuring environmental parameters in the form of analog measurement values, such as, in particular, temperature and/or humidity,
at least one analog / digital converter (3) for converting the analog measurement values into digital measurement data,
at least one processing unit (4) for generating at least one file (10, 11) from the digital measurement data, possibly in prepared form,
at least one storage unit (5) for storing digital data, in particular the at least one file (10, 11), and
at least one interface (6), preferably a USB interface, for transmitting at least digital data to and/or from the data logger (1), and
a power source (7),
**characterized in that**,
the processing unit (4) is also formed for generating an executable file (12) or that such executable file (12) is already stored in the data logger (1), whereas this executable file (12) is displayed upon connecting to an external computer (20) as a file (12) executable with the operating system (21) of the external computer (20) or is automatically executed by this operating system (21) and
is formed in such a manner that, when executing this file (12) through an e-mail client installed on the external computer (20), an e-mail is generated on the external computer (20).

2. Data logger according to claim 1, characterizedn in that with the e-mail, at least one of the following actions is automatically carried out from the data stored in the data logger (1):
- insertion of a recipient's address in the e-mail,
- insertion of a sender's address in the e-mail,
- insertion of a subject in the e-mail,
- insertion of content in the e-mail, and/or
- attachment of at least one file (10, 11) from the specified storage unit (5) of the data logger (1).

3. Data logger according to claim 1 or 2, **characterized in that** the executable file (12) is formed in such a manner that, upon executing this file (12), at least one file (10, 11) from the specified storage unit (5) of the data logger (1) is stored in an external storage medium (25).

4. Data logger according to claim 3, **characterized in that** the executable file (12) is formed in such a manner that it initiates the creation and sending of an e-mail to a predefined recipient, with which the recipient is informed of the storage of the at least one file (10, 11) from the specified storage unit (5) of the data logger (1) in the external storage medium (25).

5. Data logger according to one or more of the preceding claims, **characterized in that** the processing unit (4) is capable of storing digital data, in particular digital data in raw or processed form, in the storage unit (5) in the form of a PDF file (10) and in a file (11) with a different format, for example, as an ED3 file, whereas at least one of these files (10, 11) is provided for attaching to the specified e-mail, preferably automatically upon executing the specified executable file (12), or for storing it in an external storage medium (25).

6. Data logger according to one or more of the preceding claims, **characterized in that** the processing unit (4) is formed in such a manner that it is capable of storing prepared data from the digital measurement data in the PDF file (10).

7. Data logger according to one or more of the preceding claims, **characterized in that** the processing unit (4) is formed in such a manner that it is capable of storing the digital measurement data in raw format in the file (11) of the other format.

8. Data logger according to one or more of the preceding claims, **characterized in that** the processing unit (4) can be programmed or configured by means of special evaluation and/or programming software, in particular for storing the specified e-mail recipient's address, the specified e-mail sender's address, the specified e-mail subject and/or the specified e-mail content and/or the address or the path of the specified external storage medium (25), in a storage location (13) of the data logger (1).

9. Data logger according to one or more of the preceding claims, **characterized in that** the data logger (1) features an indicator, in particular a display, an operating element, in particular a key and/or a switch, and/or a combined indicator / operating unit (8), in particular a touchscreen.

## Revendications

1. Enregistreur de données (1) pour la saisie, la mémorisation, l'évaluation, la présentation et/ou la lecture de données de mesure, avec au moins les unités fonctionnelles suivantes logées de préférence au sein d'un coffret commun :
au moins une unité de capteur (2) pour mesurer des paramètres d'environnement sous forme de valeurs de mesures analogiques, telles qu'en particulier la température et/ou l'humidité,
au moins un convertisseur analogique/numérique (3) pour convertir les valeurs de mesure analogiques en valeurs de mesures numériques,
au moins une unité de processeur (4) pour générer au moins un fichier (10, 11) à partir des valeurs de mesure numériques, le cas échéant sous forme mise en forme,
au moins une unité de mémoire (5) pour la mémorisation de données numérique, en particulier de l'au moins un fichier (10, 11), et
au moins une unité d'interface (6), de préférence une interface USB, pour la transmission d'au moins des données numériques vers et/ou depuis l'enregistreur de données (1), et
une source d'alimentation en énergie (7),
**caractérisé en ce que**
l'unité de processeur (4) se présente en outre sous une forme pour générer un fichier exécutable (12) ou qu'un tel fichier exécutable (12) est déjà mémorisée dans l'enregistreur de donnée (1),
sachant que ce fichier exécutable (12), à la connexion avec un ordinateur externe (20) aussi bien qu'avec le système d'exploitation (21) de l'ordinateur externe (20), le fichier exécutable (12) est affiché ou exécuté automatiquement par ce système d'exploitation (21) et
qu'il se présente sous une forme telle qu'un message électronique soit généré sur l'ordinateur externe (20) lors de l'exécution de ce fichier (12) par un logiciel de messagerie électronique installé sur l'ordinateur externe (20).

2. Enregistreur de données selon la revendication 1, **caractérisé en ce que** l'une au moins des actions suivantes est automatiquement exécutée en rapport avec le message électronique à partir des données mémorisées dans l'enregistreur de données (1) :
- insérer une adresse de destinataire dans le message électronique,
- insérer une adresse d'expéditeur dans le message électronique,
- insérer un objet dans le message électronique,
- insérer un contenu dans le message électronique et/ou
- joindre au moins un fichier (10, 11) à partir de ladite unité de mémoire (5) de l'enregistreur de données (1).

3. Enregistreur de données selon la revendication 1 ou 2, **caractérisé en ce que** le fichier exécutable (12) se présente sous une forme telle qu'un fichier (10, 11) au moins depuis ladite unité de mémoire (5) de l'enregistreur de données (1) soit mémorisé sur un support d'enregistrement externe (25), lors de l'exécution de ce fichier (12).

4. Enregistreur de données selon la revendication 3, **caractérisé en ce que** le fichier exécutable (12) se présente sous une forme telle qu'il provoque la création et l'envoi d'un message électronique à un destinataire prédéfini, par lequel le destinataire est informé de la mémorisation de l'au moins un fichier (10, 11) à partir de ladite unité de mémoire (5) de l'enregistreur de donnée (1), sur ledit support d'enregistrement externe (25).

5. Enregistreur de données selon l'une ou plusieurs des revendications précédentes, **caractérisé en ce que** l'unité de processeur (4) est capable de mémoriser des données numériques, en particulier des données de mesure numériques sous forme brute ou mise en forme, sur le dispositif de mémoire (5) sous forme de fichier au format PDF (10) et dans un fichier (11) avec un format différent, par exemple un fichier ED3, sachant que l'un au moins de ces fichiers (10, 11) est prévu pour être joint audit message électronique, de préférence automatiquement lors de l'exécution dudit fichier exécutable (12), ou pour être mémorisé sur un support d'enregistrement externe (25).

6. Enregistreur de données selon l'une ou plusieurs des revendications précédentes, **caractérisé en ce Que** l'unité de processeur (4) se présente sous une forme telle qu'elle soit capable de mémoriser dans le fichier PDF (10) les données mises en forme à partir des données de mesure numériques.

7. Enregistreur de données selon l'une ou plusieurs des revendications précédentes, **caractérisé en ce que** l'unité de processeur (4) se présente sous une forme telle qu'elle soit capable de mémoriser dans le fichier (11) du format différent les données de mesure numériques au format brut.

8. Enregistreur de données selon l'une ou plusieurs des revendications précédentes, **caractérisé en ce que** l'unité de processeur (4) est programmable et/ou configurable à l'aide d'un logiciel d'analyse et/ou de programmation spécial, en particulier pour la mémorisation de ladite adresse de destinataire de message électronique, de ladite adresse d'expéditeur de message électronique, dudit objet de message électronique et/ou dudit contenu de message électronique et/ou de l'adresse ou du chemin dudit support de mémorisation externe (25) dans un emplacement de mémorisation (13) de l'enregistreur de données (1).

9. Enregistreur de données selon l'une ou plusieurs des revendications précédentes, **caractérisé en ce que** l'enregistreur de données (1) comporte un dispositif d'affichage, en particulier un écran d'affichage, un élément de commande, en particulier une touche et/ou un commutateur, et/ou un élément d'affichage/de commande combiné (8), en particulier un écran tactile.
